# EUROPEAN PATENT SPECIFICATION

(11) **EP 1 658 296 B1**
(45) Date of publication and mention of the grant of the patent: **19.12.2012**
(21) Application number: 04748601.4
(22) Date of filing: 24.05.2004
(51) Int. Cl.: C07D 501/00

(54) **CEPHEM COMPOUND**
CEPHEMVERBINDUNG
COMPOSES CEPHEM

(30) Priority: 28.05.2003 EP 03076636
(43) Date of publication of application: 24.05.2006
(73) Proprietor: DSM Sinochem Pharmaceuticals Netherlands B.V., 2613 AX Delft (NL)
(72) Inventor: VAN DEN BERG, Marco, Alexander, NL-2685 EH Poeldijk (NL); BOVENBERG, Roelof, Ary, Lans, NL-3062 DA Rotterdam (NL); RAAMSDONK, Lourina, Madeleine, Leonie, NL-2496 LN Den Haag (NL); SUTHERLAND, John David, Poynton, Cheshire SK12 1ES (GB); DE VROOM, Erik, NL-2313 JM Leiden (NL); VOLLINGA, Roeland, Christiaan, Roel, NL-3448 HC Woerden (NL)
(74) Representative: Matulewicz, Emil Rudolf Antonius
(86) International application number: PCT/NL2004/000367
(87) International publication number: WO 2004/106347

(56) References cited:
- EP-A- 0 225 128
- EP-A- 0 540 210
- US-A- 4 014 873
- US-A- 6 071 713

## Description

The present invention is concerned with novel ceph-3-em compounds and with a bioprocess for the production of these compounds. A ceph-3-em compound according to the present invention is characterised by formula [I]: 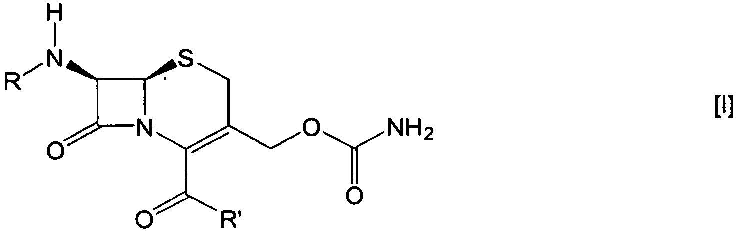 or a salt or ester thereof,
wherein R is selected from the group consisting of
a) **HOOC-X-CO-**
   wherein **X** is defined as (CH₂)₄
   or wherein **X** is **(CH₂)ₘ-CH=A-(CH₂)ₙ** or **(CH₂)ₘ-C≡C-(CH₂)ₙ,** wherein m and n each individually are 0, 1, 2 or 3 and m+n = 2 or 3, and A is CH or N,
   or wherein **X** is **(CH₂)ₚ-CH=CH-CH=C-(CH₂)_{q},** wherein **p** and **q** each individually are 0 or 1 and **p+q** = 0 or
b) **Y-CH₂-CO-** wherein **Y** is phenyl, phenoxy or tetrazolyl
   and wherein R' is selected from the group consisting of
c) OH
d) O-(alkyl 1-6C) wherein the alkyl can be straight or branched and
e) O-C(alkyl 1-6C)-O-(alkyl 1-6C) wherein the alkyl groups can be straight or branched.

This ceph-3-em compound [I] can be used as an intermediate in the production of commercial ceph-3-em antibiotics. Alternatively, this ceph-3-em compound [I] can be converted into another intermediate, namely 7-amino-3-carbamoyloxymethyl-3-cephem-4-carboxylic acid or a salt or ester thereof.

A particular advantage of this ceph-3-em compound [I] is its enhanced stability under the conditions of purification of the compound and/or the further synthesis of commercially attractive ceph-3-em antibiotics as compared to cephalosporin C. By virtue thereof the preferred compound can be isolated more easily than cephalosporin C.

Examples of commercial ceph-3-em antibiotics are cefacetril, cefaclor, cefaloglycin, cefalonium, cefaloridin, cefalotin, cefamandole, cefapirin, cefapyrin, cefatrizine, cefazedone, cefazolin, cefbuperazone, cefcapene pivoxil, cefdinir, cefditoren pivoxil, cefepime, cefixime, cefmenoxime, cefmetazole, cefminox, cefodizime, cefonicid, cefoperazone, ceforanid, cefotaxime, cefotiam, cefotiam hexetil, cefpiramide, cefpirome, cefpodoxime proxetil, cefprozil, cefroxadin, cefsulodin, ceftazidime, cefteram pivoxil, ceftezole, ceftibuten, ceftiofur, ceftizoxime, ceftriaxone, cefuroxime, cefuroxime axetil, cefuzonam.

Cefuroxime, cefoxitin and cefcapene pivoxil are examples of cephalosporin antibiotics, which share a 3-carbamoyloxymethyl group, and which cannot readily be produced from the currently available ceph-3-em intermediates, such as 7-ACA. The ceph-3-em compounds according to the present invention possess a 3-carbamoyloxymethyl group and can readily be converted into these cephalosporin antibiotics as well as in other ceph-3-em antibiotics using well-known techniques.

Cefazolin, ceftazidine and ceftriaxone are further most preferred cephalosporin antibiotics which can be prepared from a compound of formula [1] according to well known techniques.

Furthermore, the compound according to formula [I] can be used itself as an antibiotic.

A most preferred compound according to the present invention is the compound of the formula [II] (adipoyl-7-amino-3-carbamoyloxymethyl-3-cephem-4-carboxylic acid).

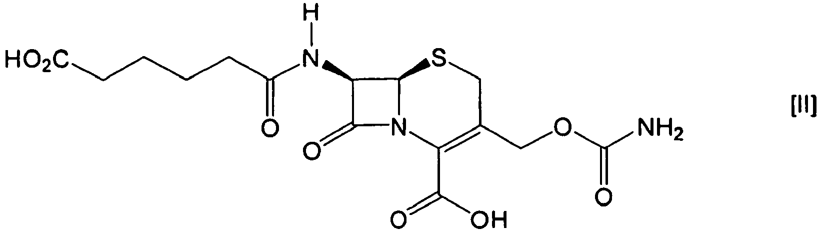

The ceph-3-em compound according to the present invention can be produced chemically according to methods known in the art, or by fermentation or by a combination of one or more biotransformation steps and one or more fermentation steps and/or one or more chemical conversion steps.

The present invention also comprises a fermentative method for the production of a ceph-3-em compound [I] as a secondary metabolite from a suitable genetically altered microorganism.

For the fermentative production of the ceph-3-em compounds according to the present invention preferably use can be made of microorganisms, which inherently possess at least part of the metabolic pathway for the production of β-lactam. For example, microorganisms can be used which possess at least part of the metabolic pathway for the production of penam or ceph-3-em β-lactam compounds. Suitable organisms for this purpose are for example fungi of the genus *Penicillium,* such as *P. chrysogenum* or of the genus *Acremonium,* such as *A. chrysogenum* or of the genus Aspergillus such as *A. nidulans,* or bacteria of the genus *Streptomyces,* such as *S. clavuligeris* or of the genus *Nocardia,* such *N. lactamdurans* or of the genus *Lysobacter* such as *L. lactamgenus.*

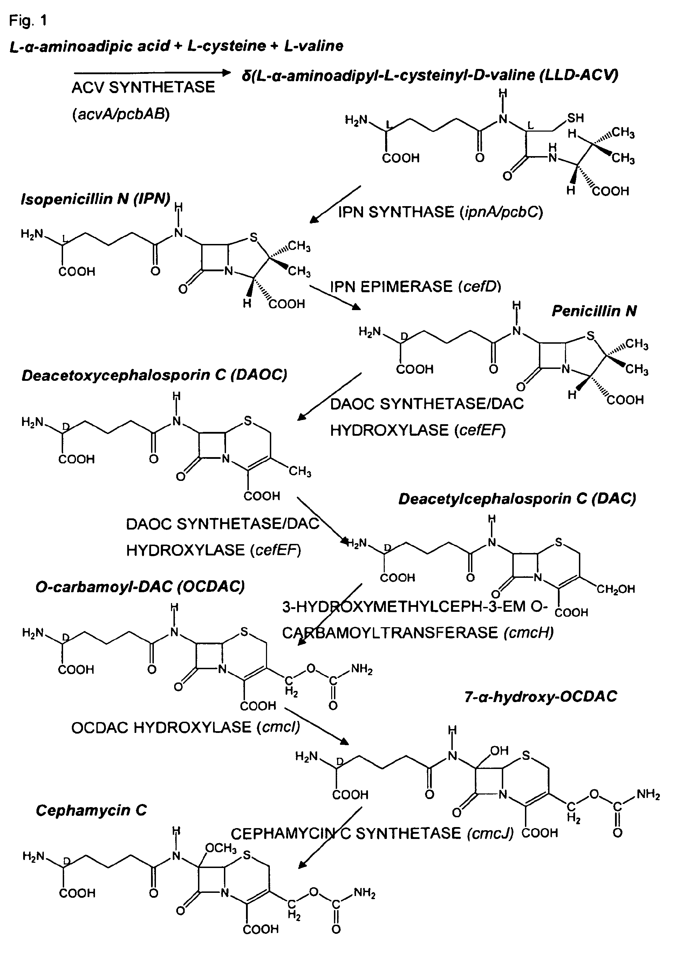

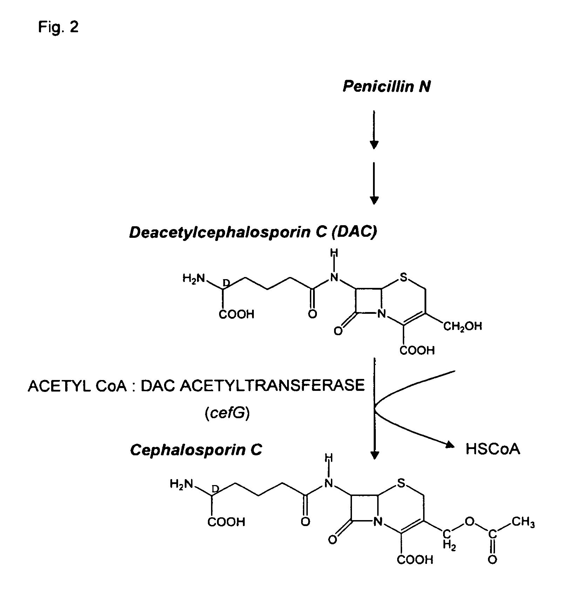

Biosynthesis of the O-carbamoylated ceph-3-em compound cephamycin C in microorganisms is believed to take place according to the scheme outlined in Fig. 1. Cephalosporin C synthesis is outlined in the scheme according to Fig.2.

Preferably, such genetically altered organisms are cultured under conditions in which in the resulting ceph-3-em compound the α-amino-adipyl side chain which is present at the 7-position in natural ceph-3-em compounds will be replaced by a desired side chain according to the present invention. To this end a composition able to deliver this side chain is supplied to the in vivo enzymatic conversion.

Suitably the *in vivo* enzymatic conversion can be supplied by a side chain precursor selected from the group consisting of
i) a compound **Y- CH₂-COOH** or a salt or ester thereof wherein **Y** is phenyl, phenoxy or tetrazolyl,
ii) a compound of the general formula **HOOC-X-COOH** or a salt or ester thereof,
   wherein **X** is defined as **(CH₂)₄**
   or wherein **X** is **(CH₂)ₘ-CH=A-(CH₂)ₙ** or **(CH₂)ₘ-C≡C-(CH₂)ₙ,** wherein m and n each individually are 0, 1, 2 or 3 and **m+n** = 2 or 3, and **A** is **CH** or **N,**
   or wherein **X** is **(CH₂)ₚ-CH=CH-CH=C-(CH₂)_{q},** wherein **p** and **q** each individually are 0 or 1 and **p+q** = 0 or 1
   If *A. chrysogenum* is used as the producing genetically engineered organism *penDE,* encoding isopenicillin N acyltransferase (Alvarez, E., B. Meesschaert, E. Montenegro, S. Gutiérrez, B. Diez, J. L. Barredo, and J. F. Martin. 1993. Eur. J. Biochem. 215:323-332) and *cmcH,* encoding carbamoyltransferase (Coque, J.J. R., F. J. Perez-Llarena, F. J. Enguita, J. L. Fuente, J. F. Martin, and P. Liras. 1995. Gene 162:21-27) should be introduced. Additionally, at least the *cef*G gene encoding DAC acetyltransferase (Felix, H.R., J. Neusch, and W. Wehrli. 1980. FEMS Microbiol. Lett. 8: 55-58; Fujisawa, Y., and T. Kanzaki. 1975. Agric. Biol. Chem. 39:2043-2048) and preferably also the *cefD1* and cefD2 gene together encoding IPN epimerase (Ullan RV, Casqueiro J, Banuelos O, Fernandez FJ, Gutierrez S, Martin JF. 2002. J Biol Chem 277(48):46216-25) should be inactivated in order to avoid undesired side products.
   If *N. lactamdurans* or *S. clavuligerus* is chosen as the producing genetically engineered organism the introduction and expression of the *penDE* is required and preferably the *cefD* gene (Jayatilake, S., J.A. Huddleston, and E.P. Abraham. 1981. Biochem. J. 195:645-647; Konomi, T., S. Herchen, J.E. Baldwin, M. Yoshida, N.A. Hunt, and A.L. Demain. 1979. Biochem. J. 184:427-430) and the *cmcI* gene encoding OCDAC hydroxylase (Xiao, X., G. Hintermann, A. Hausler, P. J. Barker, F. Foor, A. L. Demain, and J. Piret. 1993. Agents Chemother. 37:84-88) and optionally also the cmcJ gene encoding methyl transferase or cephamycin C synthetase (Coque, J.J. R., F. J. Perez-Llarena, F. J. Enguita, J. L. Fuente, J. F. Martin, and P. Liras. 1995. Gene 162:21-27), of which the latter two are the cephamycin biosynthesis late enzymes (such as described in WO95/29253) may be inactivated in order to avoid undesired side products.
   If L. *lactamgenus* is chosen as host, at least *penDE* and *cmcH* should be introduced and *cef*D preferably should be inactivated.

Insertion and inactivation of genes in organisms in order to provide the genetic make-up for the production of a compound according to the present invention can be carried out by methods known in the art. For the insertion use can be made of genomic DNA sequences or if desired use can be made of cDNA.

Preferred microorganisms for the fermentative production of the compound of the present invention are *P. chrysogenum* and *A. chrysogenum,* which by genetic engineering has been provided with DNA fragments encoding enzymes suitable for the production of the instant compound and wherein in the case of *A. chrysogenum* the appropriate genes have been inactivated.

More preferably, the fermentative production of the compound according to the present invention takes place in *P. chrysogenum* genetically engineered in a suitable way.

For this purpose a *P. chrysogenum* strain capable of producing isopenicillin N has been provided with the following set of DNA fragments:
1) DNA encoding an expandase enzyme
2) DNA encoding a hydroxylase enzyme
3) DNA encoding a O-carbamoyl transferase enzyme

Alternatively, DNA encoding the bifunctional expandase/hydroxylase enzyme may be introduced instead of DNA's encoding the separate expandase and hydroxylase enzymes, respectively.

In a preferred embodiment the invention relates to a process for the fermentative production of a compound of formula [I]: 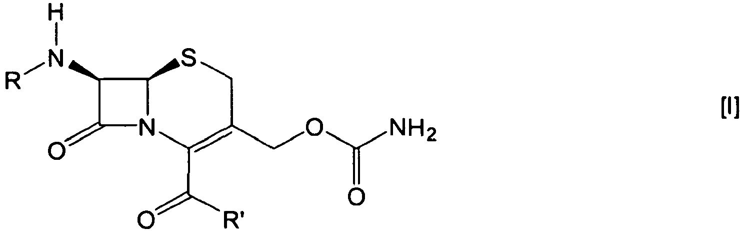 or a salt or ester thereof, wherein **R** is selected from the group consisting of
a) **HOOC-X-CO-**
   wherein **X** is defined as **(CH₂)₄**
   or wherein **X** is **(CH₂)ₘ-CH=A-(CH₂)ₙ** or **(CH₂)ₘ-C≡C-(CH₂)ₙ,** wherein m and n each individually are 0, 1, 2 or 3 and m+n = 2 or 3, and **A** is **CH** or **N,**
   or wherein **X** is **(CH₂)ₚ-CH=CH-CH=C-(CH₂)_{q},** wherein **p** and **q** each individually
   are 0 or 1 and **p+q** = 0 or
b) **Y- CH₂-CO-** wherein Y is phenyl, phenoxy or tetrazolyl
   and wherein **R'** is selected from the group consisting of
(c) OH
(d) O-(alkyl 1-6C) wherein the alkyl can be straight or branched and
(e) O-C(alkyl 1-6C)-O-(alkyl 1-6C) wherein the alkyl groups can be straight or branched.
comprising the steps of:
A) maintaining in a culture medium capable of sustaining its growth, a strain of P. *chrysogenum* which produces isopenicillin N and adding to said culture medium a feedstock comprising any one or more side chain precursors selected from the group consisting of
   i) a compound p **Y- CH₂-COOH** or a salt or ester thereof wherein Y is phenyl, phenoxy or tetrazolyl,
   ii) a compound of the general formula **HOOC-X-COOH** or a salt or ester thereof, wherein **X** is defined as **(CH₂)₄**
      or wherein **X** is **(CH₂)ₘ-CH=A-(CH₂)ₙ** or **(CH₂)ₘ-C≡C-(CH₂)ₙ,** wherein m and n each individually are 0, 1, 2 or 3 and m+n = 2 or 3, and **A** is CH or N, or wherein **X** is **(CH₂)ₚ-CH=CH-CH=C-(CH₂)_{q},** wherein **p** and **q** each individually are 0 or 1 and **p+q** = 0 or 1
      or its salts and esters which are capable of being assimilated and utilized by said strain of *P. chrysogenum* to produce a suitable acyl-6-aminopenicillanic acid (acyl-6-APA), whereby said acyl-6-APA is produced;
B) carrying out the following enzymatic conversions by in situ expression of the corresponding genes:
   i) the acyl-6-APA is *in situ* ring-expanded to form the corresponding acyl-7-amino-desacetoxycephalosporanic acid (acyl-7-ADCA) by expandase enzyme, wherein said strain of *P. chrysogenum* has been transformed by DNA encoding the expandase enzyme capable of accepting said acyl-6-APA as a substrate, whereupon as a result of its expression, said acyl-6-APA produced by said strain is also thereafter in situ ring-expanded to form the corresponding acyl-7-ADCA;
   ii) the 3-methyl side chain of said acyl-7-ADCA is in situ hydroxylated to yield the corresponding acyl-7-ADAC by hydroxylase enzyme, wherein said strain of *P. chrysogenum* has been transformed by DNA encoding the hydroxylase enzyme capable of accepting said acyl-7-ADCA as a substrate, whereupon as a result of its expression, said acyl-7-ADCA produced by said strain is also thereafter in situ hydroxylated to form the corresponding acyl-7-ADAC;
   iii) the 3-hydroxymethyl side chain of said acyl-7-ADAC is in situ O-carbamoylated to yield the compound according to formula [I] by O-carbamoyl transferase enzyme, wherein said strain of *P. chrysogenum* has been transformed by DNA encoding the O-carbamoyl transferase enzyme capable of accepting said acyl-7-ADAC as a substrate, whereupon as a result of its expression, said acyl-7-ADAC produced by said strain is also thereafter in situ carbamoylated to form a compound according to the present invention.

The DNA encoding the expandase enzyme, hydroxylase enzyme, or O-carbamoyl transferase enzyme for use according to the present invention can be obtained from micro-organisms reported to contain this DNA and which are available from culture collections or it may be obtained from micro-organisms isolated from appropriate natural sources.

Examples of microorganisms reported to contain the expandase enzyme are *A. chrysogenum* (*cefEF*)*, S. clavuligerus* (*cefE*)*, N. lactamdurans* (*cefE*)*, L. lactamgenus* (*cefE*)*.*

Examples of microorganisms reported to contain the hydroxylase enzyme are *A. chrysogenum* (*cefEF*)*, S. clavuligerus* (cefF), *N. lactamdurans* (cefF), *L. lactamgenus* (cefF).

Examples of microorganisms reported to contain the O-carbamoyl transferase enzyme are species from the Order Actinomycetes, and in particular from the Genus Streptomyces. Based on the disclosure in US Patent No. 4,075, 061, particularly suitable species which may be employed to provide the DNA encoding the desired O-transcarbamoylase activity include *S. clavuligerus* for example strain NRRL 3585 as described in British Patent No. 1,315,177; *S. wadayamensis,* for example strain ATCC 21948 as described in Dutch Patent Application No. 7308948; *S. albogriseolus,* for example strain NRRL 5735 as described in U.S. Patent No. 3,914,158; *S. lactamdurans* for example strain NRRL 3802 as described in British Patent No. 1,321,412 and S. *jumonjinensis,* for example strain NRRL 5741 as described in British Patent No. 1,387,965.

According to the disclosure of Patent Publication WO 95/29253 further suitable sources of the desired O-carbamoyl transferase encoding DNA may be *N. lactamdurans, S. lipmanii, S. panayensis, S. cattleya, S. griseus, S. todorominensis, S. filipinensis cephamicini* and *S. heteromorphus.*

Transformation of host cells, for example of *P. chrysogenum* or other fungi can, in general, be achieved by different means of DNA delivery, like PEG-Ca mediated protoplast uptake, electroporation or particle gun techniques, and selection of transformants. See for example Van den Hondel and Punt, "Gene and Transfer and Vector Development for Filamentous Fungi", in: Applied Molecular Genetics of Fungi (Peberdy, Laten, Ogden, Bennett, eds.), Cambridge University Press (1991). The application of dominant and non-dominant selection markers has been described (Van den Hondel *et al.,* supra). Selection markers of both homologous *(P. chrysogenum* derived) and heterologous (non-*P*. *chrysogenum* derived) origin have been described (Gouka et al., J. Biotechnol. 20 (1991) 189-200).

The application of various homologous or heterologous transformant selection markers, in the presence or absence of vector sequences, physically linked or not to the non-selectable DNA, in the selection of transformants is well known.

The DNA sequence encoding the expandase activity, the hydroxylase activity and the O-carbamoyl transferase activity are introduced into and expressed in this way in P. *chrysogenum,* for instance in strain Wisconsin 54-1255 (deposited at ATCC under accession number 28089). Other strains of *P. chrysogenum,* including mutants of strain Wisconsin 54-1255, having an improved beta-lactam yield, are also suitable. Examples of such high-yielding strains are the strains CBS 455.95, Panlabs P2 and ASP-78 (Barredo JL, Alvarez E, Cantoral JM, Diez B, Martin JF. 1988. Antimicrob Agents Chemother **32**(7):1061-7).

Furthermore, the *cmcH gene* together with the *cefE and cef*F *or cefEF* gene are placed under the transcriptional and translational control of heterologous or homologous control elements, preferably under control of fungal gene control elements. Those elements can be obtained from cloned fungal genes like the *P.chrysogenum* IPNS or *pcbC* gene, the β-tubulin gene, the *A. nidulans gpdA* gene, or the *A. niger glaA* gene.

As an alternative to a complete fermentative production, the compound according to the present invention can be prepared by a combination of one or more fermentative steps and one or more biotransformation steps and/or one or more chemical conversion steps.

For example, in a first step a suitable penicillin derivative, such as penicillin G or penicillin V, or cephalosporin derivative such as adipoyl-7-ADCA or adipoyl-7-ADAC can be produced fermentatively.

A suitable penicillin derivative optionally can be subjected to exchange of the 6-acyl group before conversion into the corresponding 7-acyl-3-methyl-ceph-3-em compound. The latter can in turn be converted into the corresponding 7-acyl-3-hydroxymethyl-ceph-3-em, and finally into the compound of formula [I].

For these conversions the necessary enzymes (acyltransferase, expandase, hydroxylase and carbamoyltransferase, or, alternatively, acyltransferase, exandase-hydroxylase and carbamoyltransferase, encoded by the *penDE, cefE, cef*F and cmcH genes or *penDE, cefEF* and cmcH genes, respectively) can be produced separately in any suitable host. Preferably, this is done in *Escherichia coli* according to a process known in the art. Enzymes can be purified and if needed immobilized, or used in whole cell biotransformations or crude cell-free extracts, in single and consecutive reaction vessels, or, in a one-pot reaction, to form the compound of formula [I].

Single steps in this multi-step route (e.g. penicillin G → acyl-6-APA → acyl-7-ADCA → acyl-7-ADAC → compound [I]) can be exchanged for fermentative or chemical conversion steps. E.g. the first step can be omitted if one uses adipoyl-7-ADCA produced by *P. chrysogenum* transformed with *cefE* (e.g. according to the method described in EP0523341), or alternatively, the first two steps can be omitted by starting from acyl-7-ADAC produced by *P. chrysogenum* transformed with *cefE* and *cef*F (e.g. according to the method described in EP0540210). As an alternative, chemical conversions known in the art for each single step can be applied.

### Brief description of the Figures:

- Fig. 1.:: Schematic representation of fermentative production of cephamycin C in S. *clavuligerus.*
- Fig. 2.:: Schematic representation of fermentative production of cephalosporin C in A. *chrysogenum.*
- Fig. 3.:: Plasmid used to clone the gene *cefE* and for transformation of Wisconsin 54-1225.
- Fig. 4.:: Plasmid used to clone the gene *cef*F and for transformation of Wisconsin 54-1225.
- Fig. 5.:: Plasmid used to clone the gene *cefEF* and for transformation of Wisconsin 54-1225.
- Fig. 6.:: Plasmid used to clone the gene *cmc*H and for transformation of Wisconsin 54-1225.
- Fig. 7.:: Plasmid used to clone the gene *amd*S and for transformation of Wisconsin 54-1225.

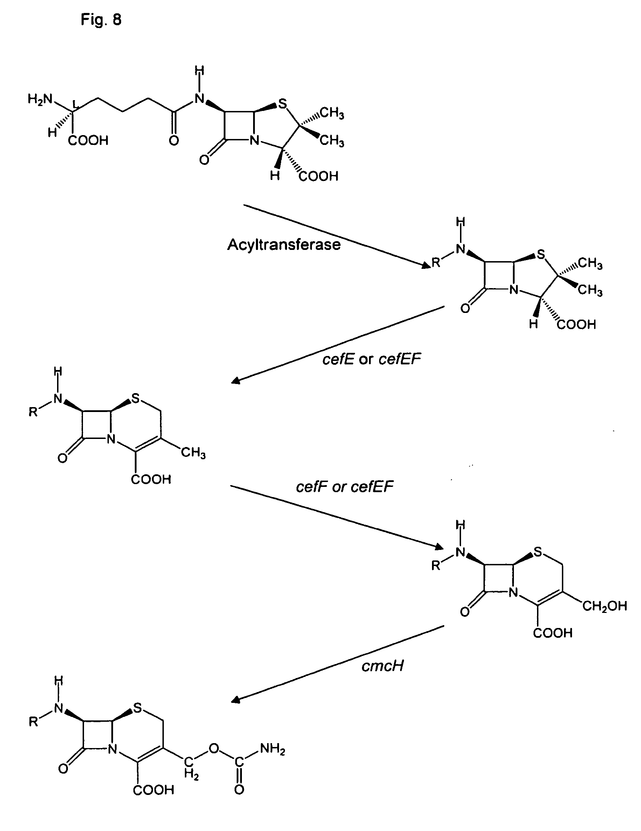

- Fig. 8.:: Schematic representation of the fermentative process according to the present invention.
- Fig. 9.:: The NMR spectrum of adipoyl-7-amino-3-carbamoyloxymethyl-3-cephem-4-carboxylic acid.
- Fig. 10.:: The NMR spectrum of the adipoyl-7-amino-3-carbamoyloxymethyl-3-cephem-4-carboxylic acid prepared according to Example 3
- Fig. 11.:: Plasmid pGK105.
- Fig. 12.:: HPLC/MS analysis of the bioconversion product by carbamoyl transferase yielding adipoyl-7-amino-3-carbamoyloxymethyl-3-cephem-4-carboxylic acid.
- Fig. 13.:: Intracellular (A) and extracellular (B) distribution of adipoyl-7-amino-3-carbamoyloxymethyl-3-cephem-4-carboxylic acid and other β-lactams (in mmol) in *P. chrysogenum* transformed according to Example 2
- Fig. 14.:: Growth inhibition by adipoyl-7-ADCA, adipoyl-ACCA and cefuroxime on *B. subtilis.*

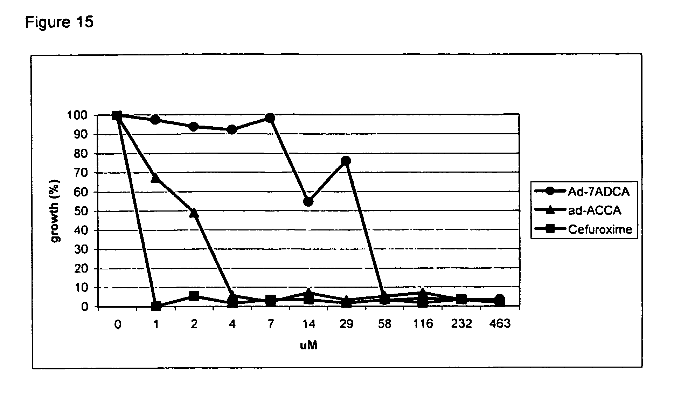

- Fig. 15.:: Growth inhibition by adipoyl-7-ADCA, adipoyl-ACCA and cefuroxime on *E. coli.*

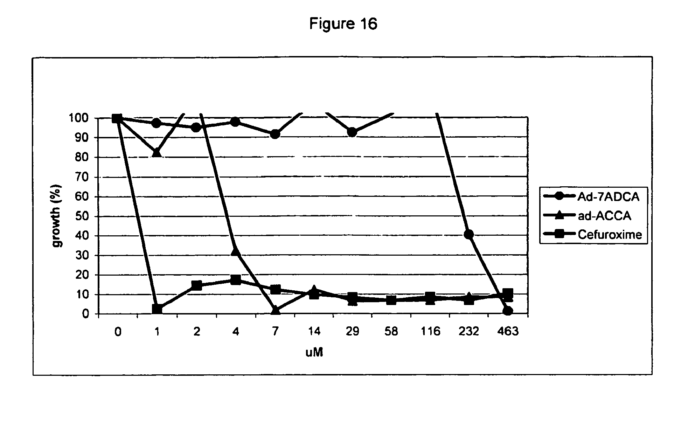

- Fig. 16.:: Growth inhibition by adipoyl-7-ADCA, adipoyl-ACCA and cefuroxime on M. *luteus.*

### EXAMPLES

### Example 1

### Transformation of a P. chrysogenum strain with the genes encoding expandase, hydroxylase and 3'-hydroxymethylcephem O-carbamoyltransferase activity

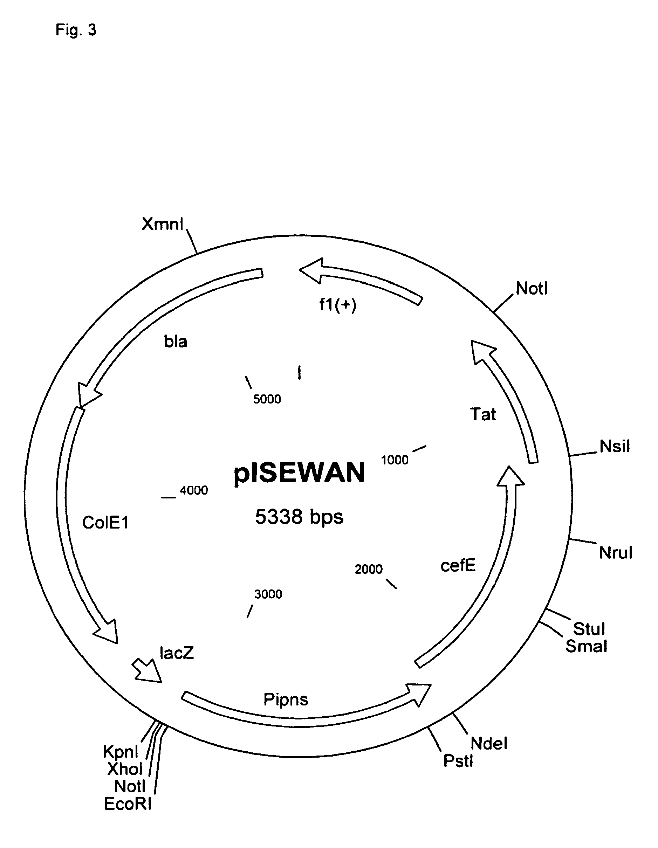

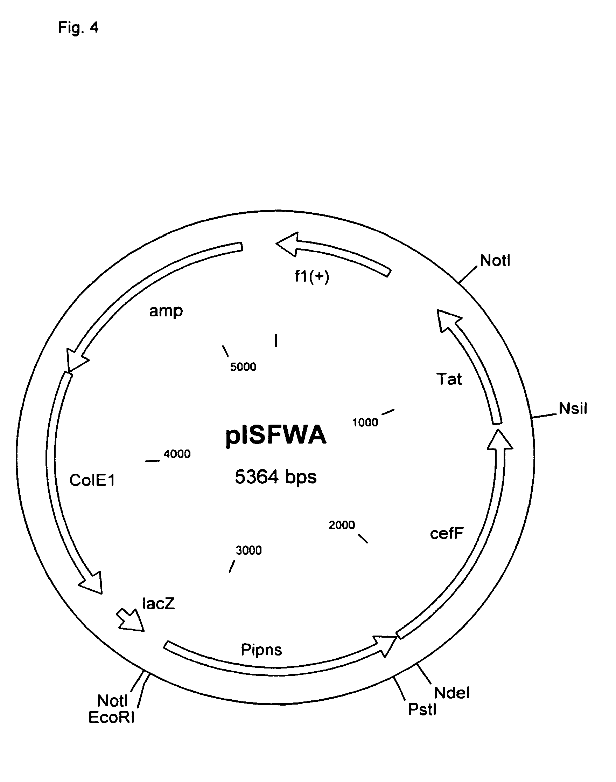

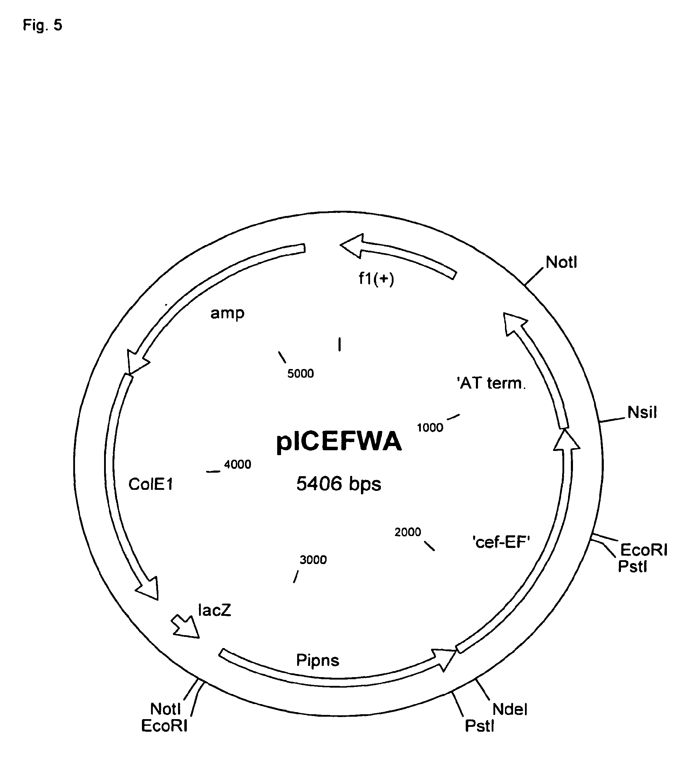

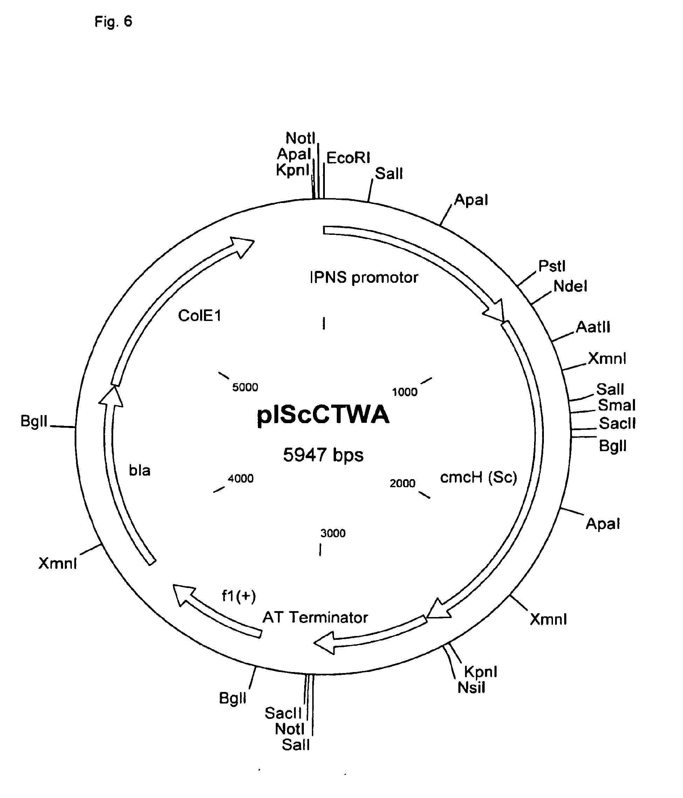

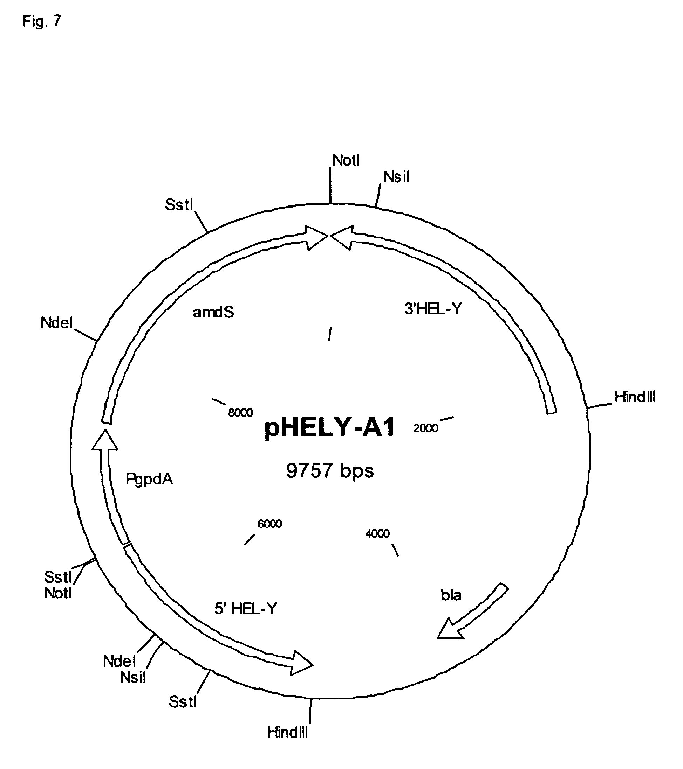

Common techniques used in gene cloning procedures are used in the present application. These techniques include polymerase chain reactions (PCR), synthetic oligonucleotide synthesis, nucleotide sequence analysis of DNA, enzymatic ligation and restriction of DNA, *E. coli* vector subcloning, transformation, and transformant selection, isolation and purification of DNA, DNA characterization by Southernblot analysis. These techniques are all very well known in the art and adequately described in many references.
Transformations were carried out with the *P. chrysogenum* strain Wisconsin 54-1255 (ATTC 28089). All constructs introduced in *P. chrysogenum* are under the control of the *P. chrysogenum* IPNS promoter and AT terminator. Other strains of *P. chrysogenum,* including mutants of strain Wisconsin 54-1255, having an improved β-lactam yield, are also suitable. An example of such a strain is CBS 455.95.
Culturing of *P. chrysogenum* for generation of protoplasts used in transformation is done in YPD- medium (1% yeast extract, 2% peptone, 2% glucose). It is well known in the art that the protoplasting and regeneration procedures may differ slightly depending on the particular strain of *P. chrysogenum* used and the transformant selection procedure applied.
*S. clavuligerus* ATCC 27064 is grown in tryptic soy broth (Difco). Chromosomal DNA of this strain is used for isolation of the *cefE* gene by PCR. The 5' forward primer 4363: 5'-GAT CAG TGA CAG TTG CAT ATG GAC ACG ACG GTG CCC ACC TTC AGC CTG-3' (SEQ ID NO. 1) and the 3' reversed primer number 4364: 5'-CCC GGG TCT AGA TCT AGA CTA TGC CTT GGA TGT GCG GCG GAT GTT-3' (SEQ ID NO. 2) were designed using the published S. *clavuligerus CefE* sequence (Kovacevic S, Weigel BJ, Tobin MB, Ingolia TD, Miller JR. J Bacteriol (1989) 171(2):754-60; Ingolia et al. U.S. Pat. No. 5,070,020).
The obtained PCR product was cut with *Nde*I and *Xba*I and the 0.9 kb fragment was ligated with pMcTNdeI also cut with *Ndel* and *Xba*I (fragment: 3.9 kb) resulting in pMcTSE. pMCTNdeI is a derivative of pMC-5 (Stanssens et al., NAR (1989) 17: 4441) and constructed by insertion of a fragment encoding the tac promoter followed by a ribosome binding sequence (RBS) site and a Ndel cloning site (see also E.P. Pat. No.0,351,029).
First the AT promoter was cloned in front of the expandase gene. The AT promoter was obtained by PCR using primer 4488: 5'-AGA ACG GAT TAG TTA GTC TGA ATT CAA CAA GAA CGG CCA GAC-3' (SEQ ID NO. 3) and 4489: 5'-GAC AGA GGA TGT GAA GCA TAT GTG CTG CGG GTC GGA AGA TGG-3' (SEQ ID NO. 4) on chromosomal DNA of *P. chrysogenum.* The oligo's were based on the *P. chrysogenum penDE* gene sequence published by Barredo et al., (1989) Gene 83:572-576 and Diez et al., Mol. Gen. Genet. (1989) 218: 572-576. The product was digested with *EcoR*I and *Nde*I resulting in a 1.5 kb fragment. This fragment was ligated with pBluescript (Stratagene) *EcoR*I*-Xba*I fragment of 3,0 kb and with pMcTSE *NdeI-XbaI* fragment of 0.9 kb containing the *S. clavuligerus* expandase gene. This results in the plasmid pASE containing the expandase gene in behind the AT promoter.
The *penDE* (encoding AT) terminator was cloned behind the expandase gene in the pASE plasmid. Therefore, chromosomal DNA of *P. chrysogenum* was used as template for the PCR of the AT terminator using 5' forward primer 4579: 5'-TTC GAT GTC AGC CTG GAC GGC GAG ACC GCC ACG TTC CAG GAT TGG ATC GGG GGC AAC TAC GTG AAC ATC CGC CGC ACA TCC AAG GCA TGA AGG CTC TTC ATG ACG-3' (SEQ ID NO. 6) and 3' reversed primer 4507: 5'-GGA CTA GTG TCG ACC CTG TCC ATC CTG AAA GAG TTG (SEQ ID NO. 5). The fragment was digested with *Bgl*I-*Spe*I and the 0.6 kb product was ligated with the plasmid pASE digested with Spel and *Bgl*I resulting in pASEWA.
Finally, the expandase gene was put behind the *P. chrysogenum* IPNS promoter (the IPNS promoter replacing the AT promoter). The IPNS promoter was amplified using P. *chrysogenum* chromosomal DNA as a template with 5' oligo 4923: 5'-CGA GGG GAA TTC CTT ATA CTG GGC TG CTG CAT TGG TCT G (SEQ ID NO. 7) (using the sequence published by: Diez,B., Gutierrez,S., Barredo,J.L., van Solingen,P., van der Voort,L.H. and Martin,J.F. (1990) J. Biol. Chem. 265 (27), 16358-16365) and 3' oligo 4924: 5'-CCC GGG CAT ATG CAT ATG GGT GTC TAG AAA AAT AAT GGT GAA AAC (SEQ ID NO. 8) (using the sequence published by Carr LG, Skatrud PL, Scheetz ME 2nd, Queener SW, Ingolia TD. (1986) Gene 48(2-3):257-66) pASEWA is digested with *EcoR*I and Ndel resulting in a 4.6 kb fragment with the expandase gene and the AT terminator was ligated with the 0.9 kb IPNS promoter PCR product digested with *Eco*RI-*Nde*I. This yields pISEWA. The *cefE* gene with the IPNS promoter and the AT terminator was obtained from pISEWAn by a *Not*I digestion (fig.3).
The hydroxylase gene (*cefF*) was isolated from *S. clavuligerus* (ATCC 27064). Chromosomal DNA of this strain was used for isolation of the *cefF* gene by PCR introducing a Ndel site upstream of the gene and a *Nsi*I site downstream. This construct was ligated behind the IPNS promoter and in front of the AT terminator resulting in pISFWA. For transformation of *P. chrysogenum* the construct including the *cefF* gene and the IPNS prmoter and AT terminator was isolated from pISFWA (Fig. 4) after cutting with *Not*I.
The A. *chrysogenum* (= *Cephalosporium acremonium)* expandase/hydroxylase gene (*cefEF*)*,* was obtained from chromosomal DNA by PCR. The designed forward oligo introduced a Ndel site and the reversed oligo a Nsil site. After digestion with Ndel and *Nsi*I the gene could be placed in the *Penicillium* expression vector resulting in pICEFWA (fig. 5). For transformation of *Penicillium,* the *cefEF* from *A. chrysogenum* was isolated from pICEFWA with the IPNS promoter and AT terminator by a *Not*I digestion.
The *cmcH* gene was obtained from *S. clavuligerus* by PCR on chromosomal DNA with the forward primer: 5'-ACA GAC CAT ATG CTC GTC GTT GCA TTC AAG -3' (SEQ ID NO. 9) and the reversed primer: 5'-GAC GGC ATG CAT TCA GGA ACC GGC TAT TCG C-3' (SEQ ID NO. 10) and was cut with *Nde*I, *Nsf*I and ligated with the *Nde*I*, Nsi*I fragment of pISEWAN (replacing the expandase gene for the *cmcH* gene) yielding pIScCTWA (Fig. 6). The *cmcH* gene including the IPNS promoter and the AT terminator was isolated from pIScCTWA by a *Not*I digest.
The *AmdS* fragment with flanks of HelY was isolated from pHELY-A1 by a digestion with *Hind*III (fig. 7).
The *cmcH,* with *cefEF* or *cefE* and *cefF* constructs were introduced by co-transformation with the *amdS* selection marker in *P. chrysogenum* ATCC 28089. The integration of the *amdS* marker enables the *P. chrysogenum* transformants to grow on selection medium containing acetamide as the sole nitrogen source.
Techniques involved in the transfer of DNA to protoplasts of *P. chrysogenum* are well known in the art and are described in many references, including Finkelstein and Ball (eds.), Biotechnology of filamentous fungi, technology and products, Butterworth-Heinemann (1992); Bennett and Lasure (eds.) More Gene Manipulations in fungi, Academic Press (1991); Turner, in: Pühler (ed), Biotechnology, second completely revised edition, VHC (1992). The Ca-PEG mediated protoplast transformation is used as described in EP635574.

### Example 2

### Fermentative production of adipoyl-7-amino-3-carbamoyloxymethyl-3-cephem-4-carboxylic acid

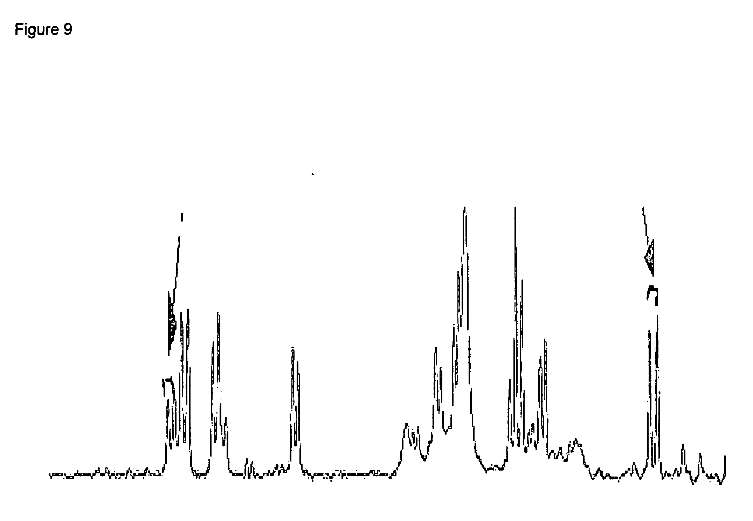

The *P. chrysogenum* transformants obtained according to Example 1 were inoculated at 2x10⁶ conidia/ml into a medium as described for penicillinV production tests in US20020039758, but instead of potassium phenoxyacetate it was supplemented with 0.5-10 mg/ml sodium adipate as a side chain precursor (pH before sterilization 5.5-6.0). The incubation took place for 144 - 169 hours at 25°C and 280 rotations per minute. Filtrates of well grown cultures were analysed by HPLC and NMR for the production of adipoyl-7-amino-3-carbamoyloxymethyl-3-cephem-4-carboxylic acid. The NMR spectrum shows the peaks characteristic for the desired compound (See figure 9.

### Example 3

### Chemical synthesis of adipoyl-7-amino-3-carbamoyloxymethyl-3-cephem-4-carboxylic acid

### PREPARATION OF PHENYLACETYL-7-AMINO-3-HYDROXYMETHYL-3-CEPHEM-4-CARBOXYLIC ACID -BENZHYDRYL ESTER [IV]

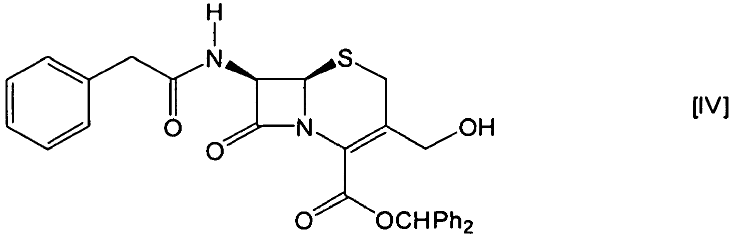

8.5 ml of 20% NaOH solution was added dropwise to a stirred solution of 7-aminocephalosporanic acid (5 g, 18.3 mmol) in water (20 ml) at 0°C. After stirring for 5 min the pH was adjusted to 8.5 with acetic acid and the solution was diluted with acetone (20 ml). Phenylacetyl chloride (2.9 ml, 22 mmol) in acetone (3 ml) was then added dropwise with the pH kept between 7.5 and 8.5 by addition of aqueous NaOH. The solution was then stirred for 1 h at 0°C. The acetone was then removed *in vacuo* before addition of ethyl acetate (70 ml) and acidification of the aqueous phase to pH 3 with dilute HCl. The organic phase was separated and the aqueous phase re-extracted with another portion of ethyl acetate. The organic phases were combined, washed with brine, (MgSO₄) and filtered. To this solution was then added a solution of diphenyldiazomethane (5 g, 25.8 mmol) in ethyl acetate (5 ml) with stirring. The solution was concentrated to ca. 40ml *in vacuo* and left overnight at 4°C. The resultant precipitate was collected by filtration and washed with ethyl acetate giving the product as a white powder (3.83 g, 40%). *δ*_{H} ((CD₃)₂SO, 300 MHz) 3.54 & 3.63 (2H, ABq, *J* 13.9, PhCH₂), 3.65 (2H, s, SCH₂), 4.25 (2H, s, CH₂OH), 5.15 (1H, d, *J* 4.7, CHCHS), 5.76 (1H, dd, *J* 4.7, 8.2, NHCH). 6.94 (1H, s, CHPh₂) 7.2-7.6 (15H, m, Ph₂CH, PhCH₂), 9.17 (1H, d, *J* 8.2, NHCH). M/z (ES+) 537 (M+Na, 100%).

### PREPARATION OF TRICHLOROACETYL-PHENYLACETYL-7-AMINO-3-CARBAMOYLOXYMETHYL-3-CEPHEM-4-CARBOXYLIC ACID -BENZHYDRYL ESTER [V]

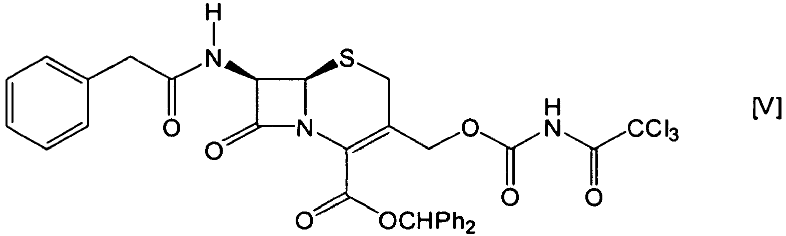

Trichloroacetyl isocyanate (0.23 ml, 1.9 mmol) was added to a stirred solution of [IV] (600 mg, 1.2 mmol) in acetone (20 ml). After stirring for 2 h a white precipitate was collected by filtration, washed with acetone and dried (811 mg, 99%). *δ*_{H} ((CD₃)₂SO, 300 MHz) 3.54 & 3.62 (2H, ABq, *J* 13.9, PhCH₂), 3.66 & 3.76 (2H, ABq, *J* 18.5, SCH₂), 4.90 & 5.02 (2H, ABq, *J* 12.8, CH₂O), 5.20 (1H, d, *J* 4.8, CHCHS), 5.82 (1H, dd, *J* 4.8, 8.1, NHCHCH), 6.96 (1H, s, CHPh₂), 7.2-7.6 (15H, m, CHPh₂, PhCH₂), 9.17 (1H, d, *J* 8.1, NHCH), 12.0 (1H, s, CONHCO). M/z (ES-) 702 (M-1,100%).

### PREPARATION OF TRICHLOROACETYL-PHENYLACETYL-7-AMINO-3-CARBAMOYLOXYMETHYL-3-CEPHEM-4-CARBOXYLIC ACID [VI]

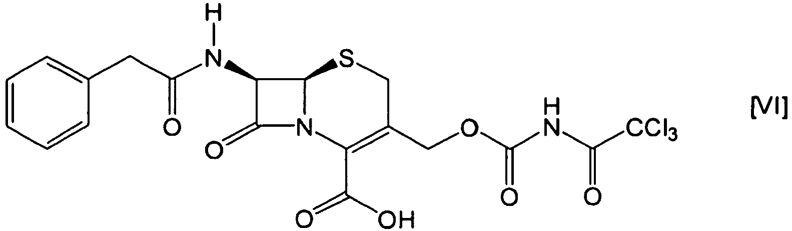

[V] (5.15 g, 7.33 mmol) was dissolved in a cooled (0°C) mixture of trifluoroacetic acid (35 ml) and anisole (4 ml). After stirring for 2 h the solution was concentrated *in vacuo* to an oil which was triturated with petroleum ether (40-60°C fraction) then dissolved in ethyl acetate (30 ml) and decolourised with charcoal. After filtering, the solution was concentrated *in vacuo* to a yellow oil which was used for the next step without further purification. *δ*_{H} ((CD₃)₂SO, 300 MHz) 3.50-3.75 (4H, m, PhCH₂, SCH₂), 4.94 (1H, part ABq, J 12.5, CH₂O), 5.14 (2H, m, part ABq CH₂O, CHCHS), 5.72 (1H, m, NHCHCH), 7.0-7.4 (5H, m, PhCH₂), 9.12 (1H, d, J 8.2, NHCH), 11.95 (1H, s, CONHCO). M/z (ES-) 536 (M-1, 67%).

### PREPARATION OF PHENYLACETYL-7-AMINO-3-CARBAMOYLOXYMETHYL-3-CEPHEM-4-CARBOXYLIC ACID [VII]

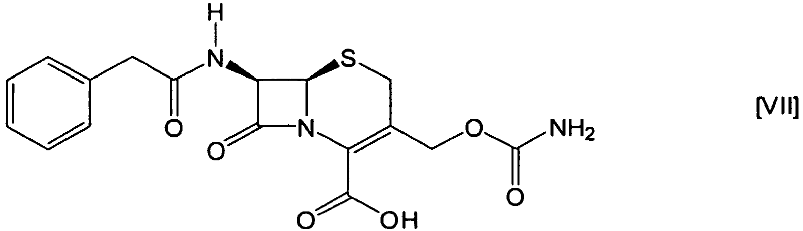

The yellow oil from the previous step was carefully dissolved by addition of 10% NaHCO₃ solution until a pH of ca. 9 was reached. The solution was then stirred overnight. The pH was then lowered to 2 using dilute HCl at which point precipitation occurred. The precipitate was removed by filtration and washed with ether to give a pale yellow solid (1.81 g, 63.3% over 2 steps). *δ*_{H} ((CD₃)₂SO, 300 MHz) 3.4-3.65 (peaks masked by H₂O Peak), 4.63 & 4.91 (2H, ABq, *J* 12.8, CH₂O), 5.10 (1H, d, *J* 4.5, CHCHS), 5.68 (1H, dd, *J* 4.5, 8.2, NHCHCH), 7.28 (5H, m, PhCH₂), 9.11 (1H, d, *J* 8.2, NHCH). δ_{H}((CD₃)₂SO + D₂O, 300 MHz) 3.52 (4H, m, PhCH₂, SCH₂), 4.63 & 4.88 (2H, ABq, *J* 12.9, CH₂O), 5.05 (1H, d, *J* 4.8, CHCHS), 5.65 (1H, d, *J* 4.7, NHCHCH), 7.28 (5H, m, PhCH₂). M/z (ES-) 390 (M-1, 20%).

### PREPARATION OF 7-AMINO-3-CARBAMOYLOXYMETHYL-3-CEPHEM-4-CARBOXYLIC ACID [VIII]

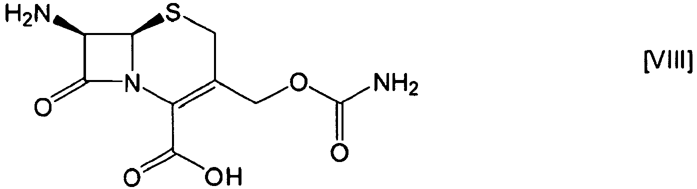

[VII] (760 mg, 1.95 mmol) was stirred in potassium phosphate buffer (20 ml, 0.5 M, pH 7) and the pH was raised to pH 7.8 by addition of aqueous NaOH. Penicillin amidase on acrylic beads (ca. 375 mg after washing to remove glucose stabilizing agent) was added and the resultant suspension was stirred for 2.5 h. The beads were then removed by filtration and the pH of the solution was lowered to 3 by addition of dilute HCl. The solution was then cooled overnight at 4°C and then filtered to yield the product as a pale yellow powder (334 mg, 63%). *δ*_{H} ((CD₃)₂SO + D₂O, 300 MHz) 3.36 & 3.53 (2H, ABq, *J* 18.1, SCH₂), 4.60 & 4.82 (2H, ABq, *J* 12.8, CH₂O), 4.74 (1H, d, *J* 4.9, CHCHS), 4.94 (1H, d, *J* 4.9, H₂NCHCH).

### PREPARATION OF CYCLIC ADIPIC ANHYDRIDE [IX]

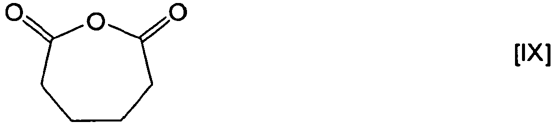

A mixture of adipic acid (5 g, 34 mmol) and acetic anhydride (15 ml) was heated at reflux for 4 h. The solution was then concentrated *in vacuo* and the remaining residue was vacuum distilled to give a colourless oil (exposure to atmospheric moisture causes polymerisation). *δ*_{H} (CDCl₃, 300 MHz) 2.0 (4H, m, CH₂CH₂CH₂CH₂), 2.76 (4H, t, *J* 6.6, OCH₂CH₂CH₂CH₂O).

### PREPARATION OF ADIPOYL-7-AMINO-3-CARBAMOYLOXYMETHYL-3-CEPHEM-4-CARBOXYLIC ACID [X]

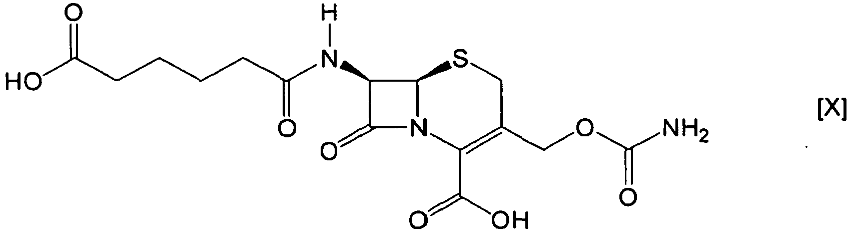

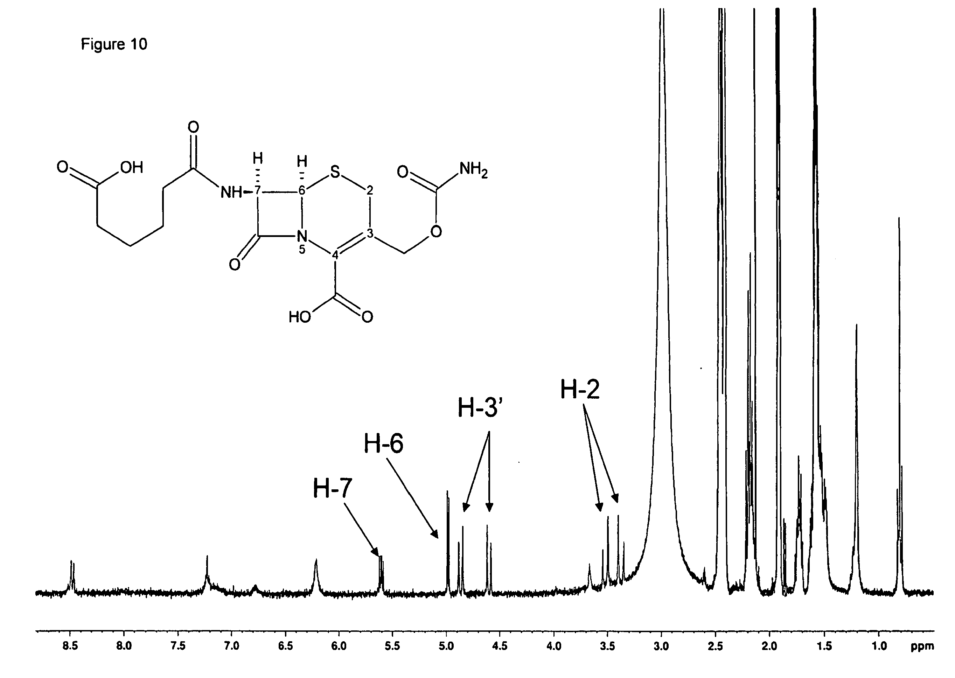

A solution of [VIII] (100 mg, 0.37 mmol) in aqueous acetone (10 ml, 1:1 v/v) was adjusted to pH 8.5 by careful addition of aqueous NaOH. To this solution was added dropwise at 0°C a solution of [IX] (80 mg, 0.625 mmol) in acetone (2 ml) with the pH kept between 7.5 and 8.5 by addition of aqueous NaOH. The resultant solution was stirred at 0°C for 2 h before removal of the acetone *in vacuo* and adjustment of the pH to 2 by addition of aqueous HCl. The solution was extracted with cyclohexanone (2 x 20 ml) and the combined organic phases concentrated to a few ml. The concentrated cyclohexanone solution was poured into cyclohexane (200 ml) giving a precipitate that was collected by filtration. (61 mg, 41%). *δ*_{H} ((CD₃)₂SO, 300 MHz) 1.50 (4H, m, CH₂CH₂CH₂CH₂), 2.22 (4H, m, CH₂CH₂CH₂CH₂), 3.45 & 3.59 (2H, ABq, *J* 18.1, SCH₂), 4.62 & 4.90 (2H, ABq, *J* 12.9, CH₂O), 5.10 (1H, d, *J* 4.8, CHCHS), 5.67 (1H, dd, *J* 4.8, 8.2, NHCHCH), 8.82 (1H, d, *J* 8.2, NHCH). M/z (ES-) 801 (2M-1, 17%), 400 (M-1, 35%) (Figure 10).

### Example 4

### In vitro production of adipoyl-7-amino-3-carbamoyloxymethyl-3-cephem-4-carboxylic acid using bioconversion

### Preparation of cell-free extract

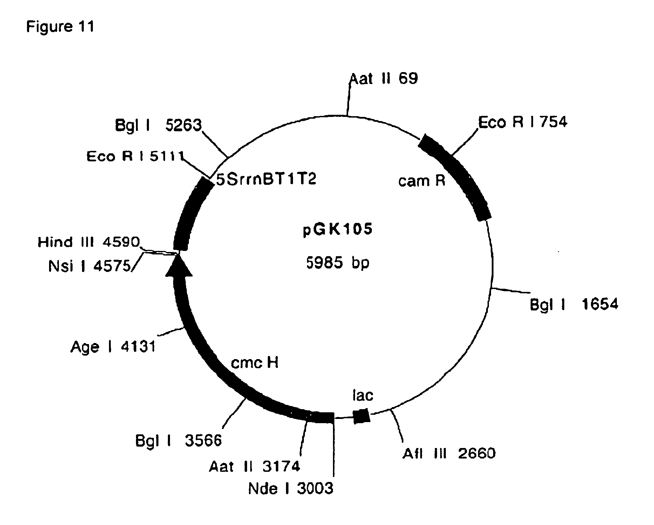

The *cmcH* gene of *Streptomyces clavuligerus* was expressed in *E. coli* XL1-Blue under the control of the *lac* promoter using plasmid pGK105 (Figure 11). For expression of the *cmcH* gene, 5 ml of chloramphenicol-containing LB growth medium was inoculated with a single colony of XL1-Blue:pGK105 and grown at 37°C for 16 hours with shaking at 250 rpm. This culture was used as a 1% inoculum for a flask containing 100 ml of sterile LB medium supplemented with chloramphenicol which was then incubated at 27°C with shaking at 250 rpm and good aeration until the OD₆₀₀ reached 0.4-0.6. IPTG was then added to the culture to a final concentration of 0.3 mM and growth was continued for a further 16 hours. Cells were harvested by centrifugation (4000 g, 20 min, 4°C) and the cell pellet was resuspended in 5 ml buffer (20 mM Tris.HCl, 200 mM NaCl, 1 mM EDTA, pH 7) and frozen either at -80°C for 1 hour or at -20°C overnight. After thawing in an ice-water bath, the cell suspension was sonicated (MSE Soniprep 150). 15-20 Cycles of 10 s sonication each followed by 10 sec. of cooling time were sufficient for complete lysis as monitored by release of soluble protein using Bradford assay. Cell debris, insoluble protein and unlyzed cells were pelleted by centrifugation (4000 g, 60 min, 4°C). The supernatant was treated with potassium clavulanate (5 mgml⁻¹) for 1 hour at 27 °C to inactivate any chromosomally encoded β-lactamases. Excess clavulanate was removed by gel filtration over Sephadex G-25® using PD-10 desalting columns and the resulting enzyme solution was used immediately or stored at -20°C until required.

### Carbamoyl transferase assay

Assays (100 µl) were performed at 28°C for four hours and contained adipoyl-deacetylcephalosporanic acid (1-5 mM), MgSO₄ (0.8 mM), MnCl₂ (1 mM), imidazole (100 mM), ATP (5.4 mM), carbamoyl phosphate (9.8 mM), pH 6.75-7. The amount of protein used ranged from 10 µg to 250 µg. The enzyme solution was thawed on ice before adding to the reaction mixture, which had been allowed to reach 28°C in a heating block. The reaction was terminated by the addition of ice-cold methanol (100 µl) and mixed before centrifuging (10000 g, 5 min, 4°C) to pellet the precipitated protein. Soluble protein that had been denatured by heat-treating at 100°C for 5 min was used in a control reaction, which was always performed alongside normal assays.

### HPLC/MS analysis

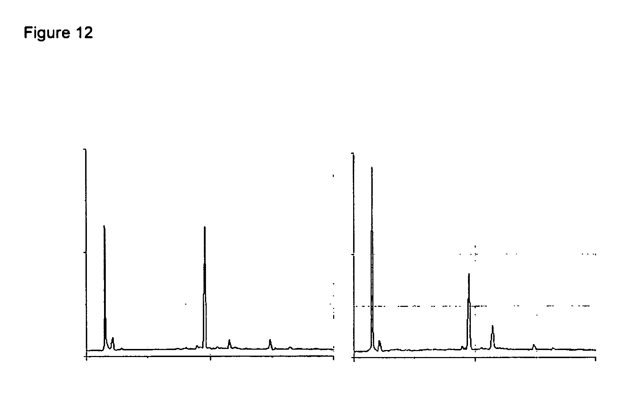

Separation of reaction components was achieved on a Spherisorb analytical C18 column (250 x 4.6 mm) at RT using a Gilson HPLC system with a mobile phase consisting of 0.1 M sodium dihydrogen phosphate as buffer A and 0.05 M sodium dihydrogen phosphate/50% acetonitrile as buffer B, a flow rate of 1 ml/min⁻¹ and monitoring at 254 nm. Using these conditions authentic standards of the cephalosporin starting material and product had retention times of 19 min and 22.5 min respectively. Product peaks from several analytical runs were pooled, acidified to pH 1.8 and extracted with cyclohexanone. The organic phase was then back-extracted with water with the pH of the biphasic extraction mixture adjusted to 7 in between periodic shaking. The aqueous phase was lyophilised, redissolved in water and analysed by electrospray (negative ion mode) ionisation MS and the carbamoylated cephalosporin product identified from its characteristic [M - H]⁻ (Figure 12).

### Conclusion

### From this experiment it can be concluded that adipoyl-7-amino-3-carbamoyloxymethyl-3-cephem-4-carboxylic acid can be produced using bioconversion

### Example 5

### Extracellular export of adipoyl-7-amino-3-carbamoyloxymethyl-3-cephem-4-carboxylic acid

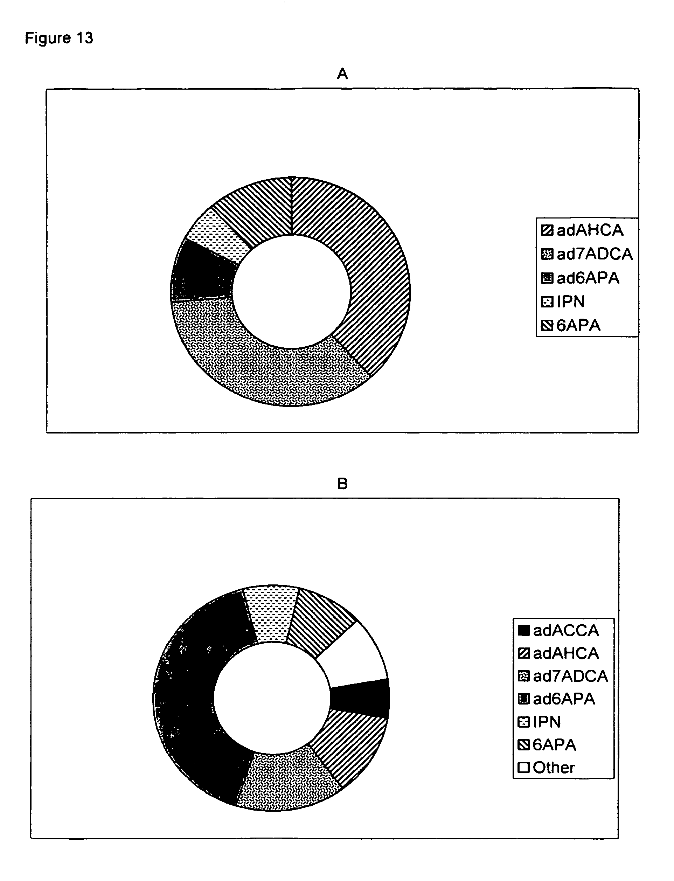

The samples as prepared according to Example 2 were analysed in more detail by separating the biomass from the filtrate. After 168 hours of growth at 25 degrees Celsius and 280 rotations per minute, the filtrates of well-grown cultures were separated from biomass via filtration and analysed by NMR. The biomass was washed twice with ice cold physiological salt (0.9 mM NaCl), frozen in liquid nitrogen, freeze dried, resuspended in water, and also analysed by NMR.
Besides adipoyl-7-amino-3-carbamoyloxymethyl-3-cephem-4-carboxylic acid the strains also produce intermediates, respectively IPN, 6APA, ad6APA, ad7ADCA and adAHCA (=adADAC). However, adipoyl-7-amino-3-carbamoyloxymethyl-3-cephem-4-carboxylic acid is the only β-lactam exclusively secreted.
The results of these experiments are summarized in Figure 13 (A and B)

### Example 6

### Fermentative production of phenoxyacetoyl-7-amino-3-carbamoyloxymethyl-3-cephem-4-carboxylic acid

The *P. chrysogenum* transformants obtained according to Example 1 were inoculated in production test medium as described in example 2, but in stead of sodium acetate it was supplemented with 5 mg/ml potassium phenoxyacetate as a side chain precursor for production tests (pH before filtration 6.0). The cultivation time was 168 hours at 25 degrees celsius and 280 rotations per minute.
Filtrates of well-grown cultures were separated from biomass and analysed by NMR. The biomass was washed twice with ice cold physiological salt (0.9 mM NaCl), frozen in liquid nitrogen, freeze dried, resuspended in water, and also analysed by NMR. Phenoxyacetoyl -7-amino-3-carbamoyloxymethyl-3-cephem-4-carboxylic acid was observed in the mycelial fractions.

### Example 7

### Fermentative production of transhydromuconoyl-7-amino-3-carbamoyloxymethyl-3-cephem-4-carboxylic acid

The *P. chrysogenum* transformants obtained according to Example 1 were inoculated in production test medium as described in example 2, but in stead of sodium acetate it was supplemented with 4 mg/ml transhydromuconic acid as a side chain precursor for production tests (pH before filtration 6.0). The cultivation time was 168 hours at 25 degrees celsius and 280 rotations per minute.
Filtrates of well-grown cultures were separated from biomass and analysed by NMR. The biomass was washed twice with ice cold physiological salt (0.9 mM NaCl), frozen in liquid nitrogen, freeze dried, resuspended in water, and also analysed by NMR. Transhydromuconoyl -7-amino-3-carbamoyloxymethyl-3-cephem-4-carboxylic acid was observed in the mycelial fractions.

### Example 8

### Fermentative production of aminoadipoyl-7-amino-3-carbamoyloxymethyl-3-cephem-4-carboxylic acid

The *P. chrysogenum* transformants obtained according to Example 1 were inoculated in production test medium as described in example 2, but without any additional side chain precursor. The cultivation time was 168 hours at 25 degrees celsius and 280 rotations per minute.
Filtrates of well-grown cultures were separated from biomass and analysed by NMR. The biomass was washed twice with ice cold physiological salt (0.9 mM NaCl), frozen in liquid nitrogen, freeze dried, resuspended in water, and also analysed by NMR. Aminoadipoyl -7-amino-3-carbamoyloxymethyl-3-cephem-4-carboxylic acid was observed in both fractions.

### Example 9

### Bioactivity of adipoyl-7-amino-3-carbamoyloxymethyl-3-cephem-4-carboxylic acid

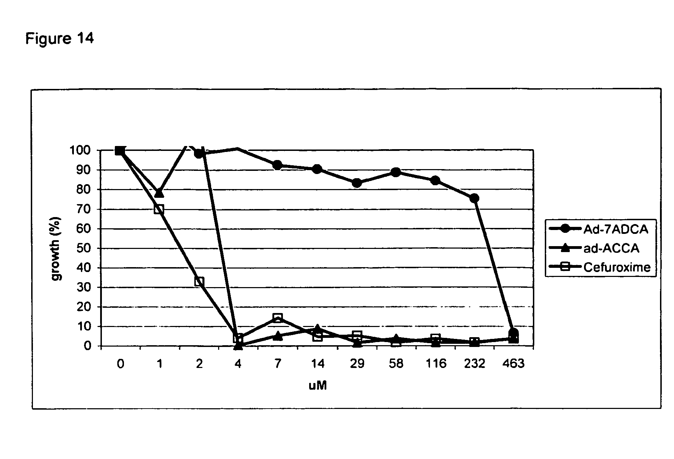

The adipoyl-7-amino-3-carbamoyloxymethyl-3-cephem-4-carboxylic acid prepared according to Example 3 was used to evaluate its activity versus different bacteria. Bacteria used were: *Escherichia coli* ESS, *Micrococcus luteus* DSM 348 [Andrade, A.C., Van Nistelrooy, J.G.M., Peery, R.B., Skatrud, P.L., De Waard, M.A., 2000, The role of ABC transporters from Aspergillus nidulans in protection against cytotoxic agents and in antibiotic production] and *Bacillus subtilis* ATCC 6633. The bacteria were grown overnight in liquid 2xTY at 37 degrees and 280 rotations per minute and subsequently diluted 1000-fold in fresh medium. Two ml deep-well microtiterplates were inoculated with 1 ml of the diluted bacterial cultures and different concentrations of adipoyl-7-amino-3-carbamoyloxymethyl-3-cephem-4-carboxylic acid (adipoyl-ACCA or Ad-ACCA) were added to individual wells wells. As control several other active and less-active b-lactams were used: 6-APA, Adipoyl-6-APA, 7-ADCA, adipoyl-7-ADCA, 7-ACA, cephalosporinC and cefuroxime. The microtiterplates were incubated for 2 days at 25 degrees Celsius and 550 rotations per minute.
The results for growth inhibition by adipoyl-7-ADCA, adipoyl-ACCA and cefuroxime are summarized in Figures 14 *(B. subtilis),* 15 *(E. coli*) and 16 *(M. luteus).*
The minimal inhibitory concentrations of adipoyl-7-amino-3-carbamoyloxymethyl-3-cephem-4-carboxylic acid for *B. subtilis* ATCC 6633, *E. coli* ESS and *M. luteus* DSM 348 were 4, 7 and 7 µM, respectively.

## Claims

1. A ceph-3-em compound **characterised by** formula [I]: 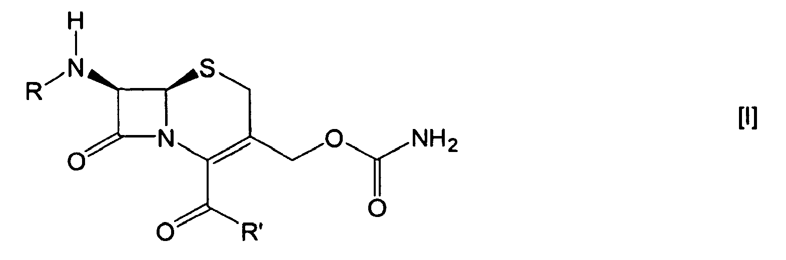 or a salt or ester thereof,
wherein R is selected from the group consisting of
a) **HOOC-X-CO-**
wherein **X** is defined as **(CH₂)₄**
or wherein **X** is **(CH₂)ₘ-CH=A-(CH₂)ₙ** or **(CH₂)ₘ-C≡C-(CH₂)ₙ,** wherein m and n each individually are 0, 1, 2 or 3 and **m+n** = 2 or 3, and **A** is **CH** or **N,**
or wherein **X** is **(CH₂)ₚ-CH=CH-CH=C-(CH₂)_{q},** wherein
p and q each individually are 0 or 1 and p+q = 0 or
b) **Y- CH₂-CO-** wherein **Y** is phenyl, phenoxy or tetrazolyl and wherein **R'** is selected from the group consisting of
c) OH
d) O-(alkyl 1-6C) wherein the alkyl can be straight or branched and
e) O-C(alkyl 1-6C)-O-(alkyl 1-6C) wherein the alkyl groups can be straight or branched.

2. Compound according to claim 1 or a salt or ester thereof, wherein the group R' is OH and wherein the group **R** is selected from adipoyl, phenoxyacetyl and tetrazoleacetyl.

3. Adipoyl-7-amino-3-carbamoyloxymethyl-3-cephem-4-carboxylic acid according to claim 1 or a salt or ester thereof.

4. A bioprocess for the fermentative production of a ceph-3-em compound **characterised by** formula [I]: 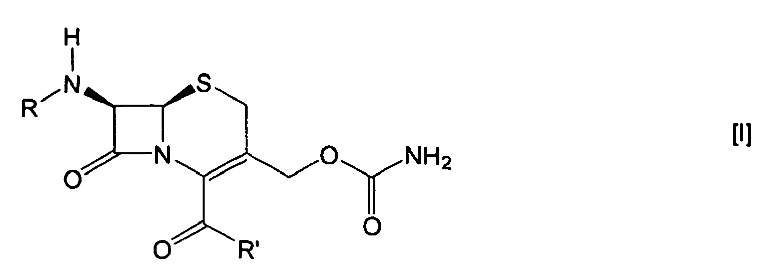 or a salt or ester thereof, wherein **R** is selected from the group consisting of
a. **HOOC-X-CO-** wherein **X** is defined as **(CH₂)₄**
or wherein **X** is **(CH₂)ₘ-CH=A-(CH₂)ₙ** or **(CH₂)ₘ-C≡C-(CH₂)ₙ,** wherein m and n each individually are 0, 1, 2 or 3 and m+n = 2 or 3, and **A** is **CH** or **N,**
or wherein **X** is **(CH₂)ₚ-CH=CH-CH=C-(CH₂)_{q},** wherein
**p** and **q** each individually are 0 or 1 and **p+q** = 0 or
b. **Y- CH₂-CO-** wherein **Y** is phenyl, phenoxy or tetrazolyl and wherein **R'** is selected from the group consisting of
c. OH
d. O-(alkyl 1-6C) wherein the alkyl can be straight or branched and
e. O-C(alkyl 1-6C)-O-(alkyl 1-6C) wherein the alkyl groups can be straight or branched.
comprising the steps of
A) maintaining in a culture medium capable of sustaining its growth, a strain of *P. chrysogenum* which produces isopenicillin N and adding to said culture medium a feedstock comprising any one or more of the side chain precursors selected from the group consisting of
● **Y- CH₂-COOH** or a salt or ester thereof wherein Y is phenyl, phenoxy or tetrazolyl
● a compound of the general formula **HOOC-X-COOH** or a salt or ester thereof, wherein **X** is defined as **(CH₂)₄**
● or wherein **X** is **(CH₂)ₘ-CH=A-(CH₂)ₙ** or **(CH₂)ₘ-C≡C-(CH₂)ₙ,** wherein **m** and **n** each individually are 0, 1, 2 or 3 and **m+n** = 2 or 3, and **A** is **CH** or **N,**
● or wherein **X** is **(CH₂)ₚ-CH=CH-CH=C-(CH₂)_{q},** wherein **p** and **q** each individually are 0 or 1 and **p+q** = 0 or 1
which are capable of being "assimilated" and utilized by said strain of *P. chrysogenum* to produce the corresponding acyl-6-aminopenicillanic acid (acyl-6-APA), whereby said acyl-6-APA is produced;
B) carrying out the following enzymatic conversion by *in situ* expression of the corresponding gene:
i) the acyl-6-APA is *in situ* ring-expanded to form the corresponding acyl-7-amino-desacetoxycephalosporanic acid (adipoyl-7-ADCA) by expandase enzyme, wherein said strain of *P. chrysogenum* has been transformed by DNA encoding the expandase enzyme capable of accepting said acyl-6-APA as a substrate, whereupon as a result of its expression, said acyl-6-APA produced by said strain is also thereafter *in situ* ring-expanded to form the corresponding acyl-7-ADCA;
ii) the 3-methyl side chain of said acyl-7-ADCA is *in situ* hydroxylated to yield the corresponding acyl-7-amino-desalkylcephalosporanic acid (acyl-7-ADAC) by hydroxylase enzyme, wherein said strain of *P. chrysogenum* has been transformed by DNA encoding the hydroxylase enzyme capable of accepting said acyl-7-ADCA as a substrate, whereupon as a result of its expression, said acyl-7-ADCA produced by said strain is also thereafter in situ hydroxylated to form the corresponding acyl-7-ADAC;
iii) the 3-hydroxymethyl side chain of said acyl-7-ADAC is *in situ* O-carbamoylated to yield the compound according to formula [I] by O-carbamoyl transferase enzyme, wherein said strain of *P. chrysogenum* has been transformed by DNA encoding the O-carbamoyl transferase enzyme capable of accepting said acyl-7-ADAC as a substrate, whereupon as a result of its expression, said acyl-7-ADAC produced by said strain is also thereafter in situ carbamoylated to form a compound according to the present invention.

5. Use of a compound according to claim 1 in the production of a ceph-3-em antibiotic.

6. Use of the compound according to claim 1 in the production of a 3-carbamoyloxymethyl-3-cephem antibiotic.

7. Use according to claim 6 wherein the 3-carbamoyloxymethyl-3-cephem antibiotic is selected from the group consisting of:
a. Cefuroxime,
b. Cefoxitine
c. Cefcapene pivoxil

8. Use of the compound according to claim 1 in the production of 7-amino-3-carbamoyloxymethyl-3-cephem-4-carboxylic acid or a salt or ester thereof.

9. Micro-organism of the species *P. chrysogenum* capable of producing isopenicillin N and suitable for the production of the compounds according to claims 1-3 and which has been provided with DNA fragments encoding :
a. an expandase enzyme
b. a hydroxylase enzyme
c. a O-carbamoyl transferase enzyme.

10. Micro-organism of the species *P. chrysogenum* capable of producing isopenicillin N and suitable for the production of the compounds according to claims 1-3 and which has been provided with DNA fragments encoding:
a. a combined expandase/hydroxylase enzyme
b. a O-carbamoyl transferase enzyme.

## Patentansprüche

1. Ceph-3-em-Verbindung, **gekennzeichnet durch** Formel [I] : 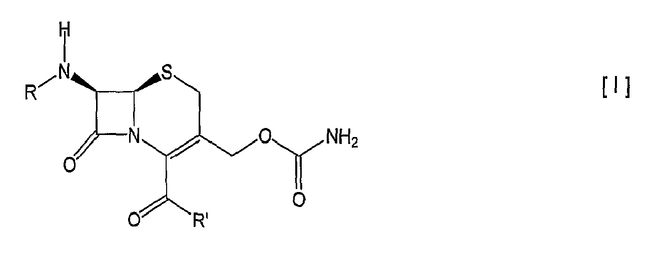 oder ein Salz oder Ester davon, wobei R aus der Gruppe ausgewählt wird, bestehend aus
a) HOOC-X-CO-,
wobei X als (CH₂)₄ definiert ist,
oder wobei X für (CH₂)ₘ-CH=A-(CH₂)ₙ oder (CH₂)ₘ-C≡C-(CH₂)ₙ steht, worin m und n jeweils einzeln 0, 1, 2 oder 3 sind und m + n = 2 oder 3 und A für CH oder N steht,
oder wobei X für (CH₂)ₚ-CH=CH-CH=C-(CH₂)_{q} steht, worin
p und q jeweils einzeln 0 oder 1 sind und p + q = 0 oder
b) Y-CH₂-CO-, wobei Y Phenyl, Phenoxy oder Tetrazolyl ist,
und wobei R' aus der Gruppe ausgewählt wird, bestehend aus
c) OH
d) 0-(Alkyl 1-6C), worin das Alkyl gerade oder verzweigt sein kann, und
e) O-C(Alkyl 1-6C)-O-(alkyl 1-6C), worin die Alkylgruppen gerade oder verzweigt sein können.

2. Verbindung nach Anspruch 1 oder ein Salz oder Ester davon, wobei die Gruppe R' für OH steht und wobei die Gruppe R aus Adipoyl, Phenoxyacetyl und Tetrazolacetyl ausgewählt wird.

3. Adipoyl-7-amino-3-carbamoyloxymethyl-3-cephem-4-carbonsäure nach Anspruch 1 oder ein Salz oder Ester davon.

4. Bioverfahren zur fermentativen Produktion einer Ceph-3-em-Verbindung, **gekennzeichnet durch** Formel [I]: 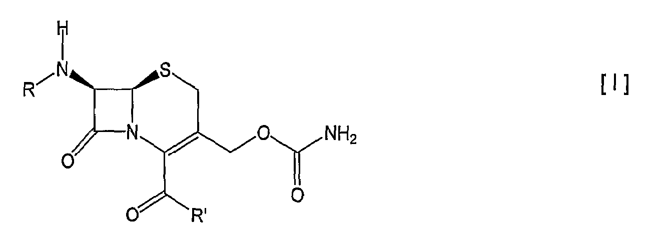 oder eines Salzes oder Esters davon, wobei R aus der Gruppe ausgewählt wird, bestehend aus
a. HOOC-X-CO-, wobei X als (CH₂)₄ definiert ist,
oder wobei X für (CH₂)ₘ-CH=A-(CH₂)ₙ oder (CH₂)ₘ-C≡C-(CH₂)ₙ steht, worin m und n jeweils einzeln 0, 1, 2 oder 3 sind und m + n = 2 oder 3 und A für CH oder N steht,
oder wobei X für (CH₂)ₚ-CH=CH-CH=C-(CH₂)_{q} steht, worin
p und q jeweils einzeln 0 oder 1 sind und p + q = 0 oder
b. Y-CH₂-CO-, wobei Y Phenyl, Phenoxy oder Tetrazolyl ist,
und wobei R' aus der Gruppe ausgewählt wird, bestehend aus
c. OH
d. 0-(Alkyl 1-6C), worin das Alkyl gerade oder verzweigt sein kann, und
e. O-C(Alkyl 1-6C)-O-(alkyl 1-6C), worin die Alkylgruppen gerade oder verzweigt sein können,
umfassend die folgenden Schritte
A) Halten eines Stammes von *P. chrysogenum,* der Isopenicillin N produziert, in einem Kulturmedium, das sein Wachstum aufrechterhalten kann, und Zufügen zu dem Kulturmedium einer Beschickung, die irgendeinen oder mehrere der Seitenkettenvorläufer umfasst, die aus der Gruppe ausgewählt werden, bestehend aus
● Y-CH₂-COOH oder einem Salz oder Ester davon, wobei Y Phenyl, Phenoxy oder Tetrazolyl ist,
● einer Verbindung der allgemeinen Formel HOOC-X-COOH oder einem Salz oder Ester davon, wobei X als (CH₂)₄ definiert ist,
● oder wobei X für (CH₂)ₘ-CH=A-(CH₂) oder (CH₂)ₘ-C≡C-(CH₂)ₙ steht, worin m und n jeweils einzeln 0, 1, 2 oder 3 sind und m + n = 2 oder 3 und A für CH oder N steht,
● oder wobei X für (CH₂)ₚ-CH=CH-CH=C-(CH₂)_{q} steht, worin p und q jeweils einzeln 0 oder 1 sind und p + q = 0 oder 1,
die in der Lage sind, von dem Stamm von *P. chrysogenum* unter Produktion der entsprechenden Acyl-6-aminopenicillansäure (Acyl-6-APA) "assimiliert" und genutzt zu werden, wodurch die Acyl-6-APA produziert wird;
B) Durchführen der folgenden enzymatischen Umwandlung **durch** in situ-Expression des entsprechenden Gens:
i) Acyl-6-APA wird *in situ* unter Bildung der entsprechenden Acyl-7-aminodesacetoxycephalosporansäure (Adipoyl-7-ADCA) mittels Expandase-Enzym Ring-expandiert, wobei der Stamm von *P. chrysogenum* mit DNA transformiert worden ist, die das Expandase-Enzym codiert, das die Acyl-6-APA als Substrat akzeptieren kann, wonach die von dem Stamm produzierte Acyl-6-APA infolge ihrer Expression anschließend auch *in situ* unter Bildung der entsprechenden Acyl-7-ADCA Ring-expandiert wird;
ii) die 3-Methyl-Seitenkette der Acyl-7-ADCA wird *in situ* unter Erhalten der entsprechenden Acyl-7-aminodesalkylcephalosporansäure (Acyl-7-ADAC) mittels Hydroxylase-Enzym hydroxyliert, wobei der Stamm von *P. chrysogenum* mit DNA transformiert worden ist, die das Hydroxylase-Enzym codiert, das die Acyl-7-ADCA als Substrat akzeptieren kann, wonach die von dem Stamm produzierte Acyl-7-ADCA infolge ihrer Expression danach auch *in situ* unter Bildung der entsprechenden Acyl-7-ADAC hydroxyliert wird;
iii) die 3-Hydroxymethyl-Seitenkette der Acyl-7-ADAC wird *in situ* unter Erhalten der Verbindung der Formel [I] **durch** O-Carbamoyltransferase-Enzym 0-carbamoyliert, wobei der Stamm von *P. chrysogenum* mit DNA transformiert worden ist, die das 0-Carbamoyltransferase-Enzym codiert, das die Acyl-7-ADAC als Substrat akzeptieren kann, wonach die **durch** den Stamm produzierte Acyl-7-ADAC infolge ihrer Expression anschließend auch *in situ* unter Bildung einer erfindungsgemäßen Verbindung carbamoyliert wird.

5. Verwendung einer Verbindung nach Anspruch 1 zur Produktion eines Ceph-3-em-Antibiotikums.

6. Verwendung einer Verbindung nach Anspruch 1 zur Produktion eines 3-Carbamoyloxymethyl-3-cephem-Antibiotikums.

7. Verwendung nach Anspruch 6, wobei das 3-Carbamoyloxymethyl-3-cephem-Antibiotikum aus der Gruppe ausgewählt wird, bestehend aus:
a. Cefuroxim,
b. Cefoxitin
c. Cefcapen-Pivoxil.

8. Verwendung der Verbindung nach Anspruch 1 zur Produktion von 7-Amino-3-carbamoyloxymethyl-3-cephem-4-carbonsäure oder eines Salzes oder Esters davon.

9. Mikroorganismus der Spezies *P. chrysogenum,* der in der Lage ist, Isopenicillin N zu produzieren, und geeignet zur Produktion der Verbindungen nach den Ansprüchen 1-3 und der mit DNA-Fragmenten ausgestattet wurde, die Folgendes codieren:
a. ein Expandase-Enzym
b. ein Hydroxylase-Enzym
c. ein O-Carbamoyltransferase-Enzym.

10. Mikroorganismus der Spezies *P. chrysogenum,* der in der Lage ist, Isopenicillin N zu produzieren, und geeignet zur Produktion der Verbindungen nach den Ansprüchen 1-3 und der mit DNA-Fragmenten ausgestattet wurde, die Folgendes codieren:
a. ein kombiniertes Expandase/Hydroxylase-Enzym
b. ein O-Carbamoyltransferase-Enzym.

## Revendications

1. Composé céph-3-ème **caractérisé par** la formule [I] : 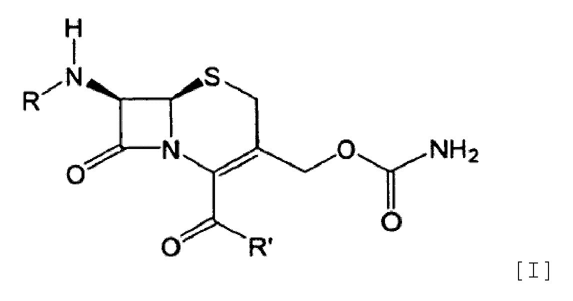 ou un sel ou ester de celui-ci,
dans lequel R est choisi dans le groupe constitué de
a) HOOC-X-CO-
dans lequel X est défini par (CH₂)₄
ou dans lequel X est (CH₂)ₘ-CH=A-(CH₂)ₙ ou (CH₂)ₘ-C≡C-(CH₂)ₙ, m et n étant chacun individuellement 0, 1, 2 ou 3 et m+n = 2 ou 3, et A est CH ou N,
ou dans lequel X est (CH₂)ₚ-CH=CH-CH=C-(CH₂)_{q}, p et
q étant chacun individuellement 0 ou 1 et p+q = 0 ou
b) Y-CH₂-CO- dans lequel Y est phényle, phénoxy ou tétrazolyle et dans lequel R' est choisi dans le groupe constitué de
c) OH
d) O-(alkyle en C₁₋₆) dans lequel l'alkyle peut être linéaire ou ramifié et
e) 0-C(alkyle en C₁₋₆)-O-(alkyle en C₁₋₆) dans lequel les groupes alkyle peuvent être linéaires ou ramifiés.

2. Composé selon la revendication 1 ou un sel ou ester de celui-ci, dans lequel le groupe R' est OH et le groupe R est choisi parmi adipoyle, phénoxyacétyle et tétrazolacétyle.

3. Acide adipoyl-7-amino-3-carbamoyloxyméthyl-3-céphème-4-carboxylique ou un sel ou ester de celui-ci.

4. Procédé biologique pour la production par fermentation d'un composé céph-3-ème **caractérisé par** la formule [I] : 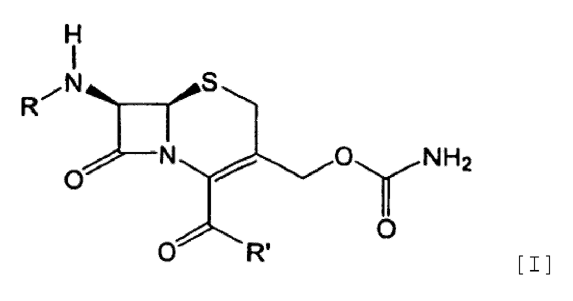 ou un sel ou ester de celui-ci, dans lequel R est choisi dans le groupe constitué de
a. HOOC-X-CO- dans lequel X est défini par (CH₂)₄
ou dans lequel X est (CH₂)ₘ-CH=A-(CH₂)ₙ ou (CH₂)ₘ-C≡C-(CH₂)ₙ, m et n étant chacun individuellement 0, 1, 2 ou 3 et m+n = 2 ou 3, et A est CH ou N,
ou dans lequel X est (CH₂)ₚ-CH=CH-CH=C-(CH₂)_{q}, p et
q étant chacun individuellement 0 ou 1 et p+q = 0 ou
b. Y-CH₂-CO- dans lequel Y est phényle, phénoxy ou tétrazolyle et dans lequel R' est choisi dans le groupe constitué de
c. OH
d. O-(alkyle en C₁₋₆) dans lequel l'alkyle peut être linéaire ou ramifié et
e. 0-C(alkyle en C₁₋₆)-O-(alkyle en C₁₋₆) dans lequel les groupes alkyle peuvent être linéaires ou ramifiés,
comprenant les étapes consistant à
A) maintenir dans un milieu de culture capable de soutenir sa croissance, une souche de *P. chrysogenum* qui produit de l'isopénicilline N et d'ajouter audit milieu de culture une matière première comprenant l'un quelconque ou plusieurs des précurseurs de chaîne latérale choisis dans le groupe constitué de
- Y-CH₂-COOH ou un sel ou ester de celui-ci dans lequel Y est phényle, phénoxy ou tétrazolyle,
- un composé de formule générale HOOC-X-COOH ou un sel ou ester de celui-ci, dans lequel X est défini par (CH₂)₄
- ou dans lequel X est (CH₂)ₘ-CH=A-(CH₂)ₙ ou (CH₂)ₘ-C≡C-(CH₂)ₙ, m et n étant chacun individuellement 0, 1, 2 ou 3 et m+n = 2 ou 3, et A est CH ou N,
- ou dans lequel X est (CH₂)ₚ-CH=CH-CH=C-(CH₂)_{q}, p et q étant chacun individuellement 0 ou 1 et p+q = 0 ou 1
qui peuvent être « assimilés » et utilisés par ladite souche de *P. chrysogenum* pour produire l'acide acyl-6-aminopénicillanique correspondant (acyl-6-APA), de telle manière que ledit acyl-6-APA soit produit ;
B) conduire la conversion enzymatique suivante par expression *in situ* du gène correspondant :
i) l'acyl-6-APA est expansé de façon cyclique in situ pour former l'acide acyl-7-amino-desacétoxycéphalosporanique (adipoyl-7-ADCA) correspondant par l'enzyme expandase, ladite souche de *P. chrysogenum* ayant été transformée par l'ADN codant pour l'enzyme expandase capable d'accepter ledit acyl-6-APA en tant que substrat, après quoi en conséquence de son expression, ledit acyl-6-APA produit par ladite souche étant ensuite en outre expansé de façon cyclique *in situ* pour former l'acyl-7-ADCA correspondant ;
ii) la chaîne latérale 3-méthyle dudit acyl-7-ADCA est hydroxylée *in situ* pour obtenir l'acide acyl-7-amino-désalkylcéphalosporanique (acyl-7-ADAC) correspondant par l'enzyme hydroxylase, ladite souche de *P. chrysogenum* ayant été transformée par l'ADN codant pour l'enzyme hydroxylase capable d'accepter ledit acyl-7-ADCA en tant que substrat, après quoi en conséquence de son expression, ledit acyl-7-ADCA produit par ladite souche étant en outre ensuite hydroxylé *in situ* pour former l'acyl-7-ADAC correspondant ;
iii) la chaîne latérale 3-hydroxyméthyle dudit acyl-7-ADAC est O-carbamoylé *in situ* pour obtenir le composé selon la formule [I] par l'enzyme 0-carbamoyle transférase, ladite souche de *P. chrysogenum* ayant été transformée par l'ADN codant pour l'enzyme O-carbamoyle transférase capable d'accepter ledit acyl-7-ADAC en tant que substrat, après quoi, en conséquence de son expression, ledit acyl-7-ADAC produit par ladite souche est également ensuite carbamoylé *in situ* pour former un composé selon la présente invention.

5. Utilisation d'un composé selon la revendication 1 dans la production d'un antibiotique céph-3-ème.

6. Utilisation du composé selon la revendication 1 dans la production d'un antibiotique 3-carbamoyloxyméthyl-3-céphème.

7. Utilisation selon la revendication 6 dans laquelle l'antibiotique 3-carbamoyloxyméthyl-3-céphème est choisi dans le groupe constitué de :
a. céfuroxime,
b. céfoxitine
c. céfcapène pivoxil

8. Utilisation du composé selon la revendication 1 dans la production d'acide 7-amino-3-carbamoyloxyméthyl-3-céphèm-4-carboxylique ou un sel ou ester de celui-ci.

9. Micro-organisme de l'espèce *P. chrysogenum* capable de produire l'isopénicilline N et adapté pour la production des composés selon les revendications 1 à 3 et qui a été pourvu de fragments d'ADN codant pour :
a. une enzyme expandase
b. une enzyme hydroxylase
c. une enzyme O-carbamoyle transférase.

10. Micro-organisme de l'espèce *P. chrysogenum* capable de produire de l'isopénicilline N et adapté pour la production des composés selon les revendications 1 à 3 et qui a été pourvu de fragments d'ADN codant pour :
a. une enzyme expandase/hydroxylase combinée
b. une enzyme O-carbamoyle transférase.
